# Europäisches Patentamt

(19) **European Patent Office**

**Office européen des brevets**

(11) Veröffentlichungsnummer: **0 163 133**

**B1**

(12)

# EUROPÄISCHE PATENTSCHRIFT

(45) Veröffentlichungstag der Patentschrift:
06.12.89

(51) Int. Cl.⁴: **B 32 B 15/08**

(21) Anmeldenummer: **85105047.6**

(22) Anmeldetag: **25.04.85**

(54) Einlegesohlen und Bestandteile von Sohlen sowie Verfahren zur Herstellung derselben.

| | |
|---|---|
| (30) Priorität: 30.05.84 DE 3420121<br>27.06.84 DE 3431474<br>30.10.84 DE 3439727 | (73) Patentinhaber: **Weisser, Peter, Dr., Bahnhofstrasse 34, CH- 9470 Buchs (CH)** |
| (43) Veröffentlichungstag der Anmeldung:<br>04.12.85 Patentblatt 85/49 | (72) Erfinder: **Latzke, Arno Walter, Mühltobel 946, CH-9429 Zelg- Wolfhalden (CH)** |
| (45) Bekanntmachung des Hinweises auf die Patenterteilung:<br>06.12.89 Patentblatt 89/49 | (74) Vertreter: **Werner, Hans- Karsten, Dr., Deichmannhaus am Hauptbahnhof, D-5000 Köln 1 (DE)** |
| (84) Benannte Vertragsstaaten:<br>**AT BE CH DE FR GB IT LI LU NL SE** | |
| (56) Entgegenhaltungen:<br>DE-U-8 130 561<br>GB-A-742 787<br>US-A-4 005 532 | |

LIBER, STOCKHOLM 1989

2

## Beschreibung

Gegenstand der vorliegenden Erfindung sind verbesserte Einlegesohlen und verbesserte Bestandteile von Sohlen sowie Verfahren zu deren Herstellung.

Einlegesohlen und Bestandteile von Sohlen sind bereits aus den verschiedensten Materialien hergestellt worden. Keines dieser Materialien hat sich als ideal erwiesen, da sie stets nur einige der erwünschten Eigenschaften aufwiesen. So sollten Einlegesohlen einerseits so dünn wie möglich sein, andererseits gut gegen Kälte und Wärme isolieren, eine gewisse Be- und Entlüftung ermöglichen, um die Schweißbildung zu unterdrücken, eine gewisse Elastizität aufweisen und dennoch guten Halt geben, auch nach längerem Tragen formstabil bleiben und mechanisch so widerstandsfähig sein, daß sie ausreichend abrieb- und verschleißfest sind.

Die vorliegende Erfindung betrifft Einlegesohlen und Bestandteile von Sohlen mit einer zwischen zwei Kunststoffschichten eingebetteten Metallschicht sowie einer hautverträglichen Außenschicht bei deren alle Schichte fest miteinander laminiert sind. Solche Einlegesohlen und Bestandteile von Sollen, bekannt an sich aus der Druckschrift GB-A-742 787, sind dadurch gekennzeichnet, daß:

- die Kunstoffschichten aus geschlossenporigem Polyethylenschaumstoff bestehen und ursprünglich - d.h. in unverpreßtem Zustand - je 2 bis 8 mm stark sind;
- die Schaumstoffschichten zur orthopädischen Formgebung mindestens im Vorderfußbereich flach verpreßt sind, wobei die Dicke der Einlegesohlen bzw. der Bestandteile von Sohlen im Vorderfußbereich geringer ist als im Mittelfuß- und Fersenbereich;
- die Metallschicht 0,02 bis 0,08 mm stark ist; und
- im Bereich der Kunststoffschicht (3) und der hautverträglichen Stoffschicht (S) eine Noppung vorgesehen ist.

In den als Prioritäten beanspruchten deutschen Patentanmeldungen P-3 420 121, P-3 431 474 und P-3 439 727 sind unter anderem Einlegesohlen vorgeschlagen worden, die aus einer 0,8 bis 8 mm starken Schaumstoffschicht, oder einer hautverträglichen Schicht aus Textil, Gewebe, Leder, Kork oder Kunststoff, einer flexiblen wärmeleitenden Metallschicht und einer 0,8 bis 8 mm starken Schaumstoffschicht bestehen, die gegebenenfalls genoppt ist.

Die orthopädische Verformung besteht insbesondere in einem seitlichen Hochziehen im Fersenbereich, einer Aufwölbung im inneren Bereich des Mittelfußes und vorzugsweise einer deutlichen Sprengung. Unter Sprengung versteht man eine S-förmige Verformung, durch die der Fersenbereich deutlich über dem Vorderfußbereich zu liegen kommt. Diese orthopädische Verformung und Sprengung führt zu besonders angenehmen Tragkomfort und gleichzeitig einem dauerhaften Anschmiegen der Einlegesohle an die Sohle des Schuhwerkes. Dies ist von besonderer Bedeutung bei Damenschuhen, insbesondere wenn diese überhöhte Absätze aufweisen. Die erfindungsgemäßen Einlegesohlen sind daher auch optimal geeignet für Pumps.

Eine Besonderheit der erfindungsgemäßen Einlegesohlen besteht darin, daß nach der orthopädischen Verformung, Verpressung und Noppung die Dicke des Laminats im Vorderfußbereich geringer ist als im Mittelfuß und Fersenbereich. Durch die stärkere Verpressung im Vorderfußbereich sinkt zwar die Wärmeisolierung, steigt jedoch der Tragkomfort. Überraschenderweise führen jedoch einerseits die Noppung auf der Oberfläche und zum anderen die Metallfolie im Zwischenbereich zu einer guten und gleichmäßigen Erwärmung des Vorderfußes, wobei sich gezeigt hat, daß überflüssige Wärme aus dem Fersen- und Mittelfußbereich in den Vorderfußbereich abgeleitet wird.

Die Noppung führt einerseits zu einer Mikrozirkulation von Luft und Feuchtigkeit und andererseits zu einem die Durchblutung fördernden Massageeffekt. Diese Effekte sind bei den erfindungsgemäß durch Wärme- und Druckeinwirkung verformten, verpressten und genoppten Einlegesohlen stärker als bei unbehandelten Laminaten. Vergleichende Messungen deuten daraufhin, daß die Verformung der Gasbläschen im geschlossenporigen Polyethylenschaumstoff unter Einwirkung von Wärme und Druck von der Kugel- zur Linsenform einen entscheidenden Einfluß hat. Weiterhin führt diese dauernde Verformung unter Wärme- und Druckeinwirkung zu einer erhöhten Formstabilität bei langfristiger Beanspruchung der Einlegesohlen, so daß deren Lebensdauer erheblich verlängert wird.

Die orthopädische Verformung erhöht nicht nur den Tragkomfort sondern auch die bessere Ableitung bzw. Umverteilung der Wärme vom Fersen- und Mittelfußbereich in den Vorderfußbereich, in welchem durch die erfindungsgemäße fläche Verpressung die Wärmeisolierung an sich verringert ist.

Die hautverträgliche Stoffschicht (S) trägt ebenfalls zum Tragkomfort bei. Sie ermöglicht darüberhinaus eine verbesserte Luft- und Feuchtigkeitsverteilung.

Die genaueren Messungen der erfindungsgemäß modifizierten Einlegesohlen haben weiter gezeigt, daß die Wärmeisolierung in dem genoppten Bereich (3) und (S) durch die stärkere Verpressung geringer ist als auf der anderen Seite der Metallfolie im Bereich (1). Dies führt dazu, daß die untere, nicht genoppte Schaumstoffschicht (1) überwiegend gegen größere Temperaturabweichungen abschirmt, d.h. im Winter gegen Kälte und im Sommer gegen Hitze. Die Metallschicht (2) führt zu einer Umverteilung lokaler Temperaturunterschiede. Die obere verpresste Schaumstoffschicht führt ebenfalls zu einer gewissen Wärmeisolierung, insbesondere zu einem Wärmeaustausch an der Fußsohle im Mikrobereich zwischen den einzelnen Noppen. Die Noppung führt insbesondere beim Be- und Entlasten des Fußes zu einem Massageeffekt, der die Durchblutung fördert.

Vergleichsmessungen mit einer handelsüblichen Einlegesohle, die aus einer hautverträglichen Stoffschicht, zwei verschiedenen Schaumstoffschichten und einer mit Aluminium bedampften Gitterfolie besteht, haben gezeigt, daß die Wärmeisolierung bei punktueller Wärmezufuhr etwa um den Faktor 10 verbessert wird. Zur Abschirmung gegen Kälte im Winter war es somit nötig, dickere Sohlen zu verwenden. Derartige Sohlen führten jedoch im Sommer zur Schweißbildung und zum Brennen der Füße. Die erfindungsgemäßen Einlegesohlen können somit erstmals im Sommer und im Winter in gleicher Stärke verwendet werden und führen das ganze Jahr hindurch zu optimalem Tragkomfort.

Der Effekt beruht insbesondere darauf, daß erfindungsgemäß erstmals ein guter Wärmeleiter mit einem guten Wärmeisolator kombiniert wird. Die Kombination dieser beiden verschiedenartigen Werkstoffe ist vom Fachmann bisher nie in Erwägung gezogen worden, da er zur Erzielung einer guten Wärmeisolierung nur Wärmeisolatoren verwendet hat und die Verwendung von gut wärmeleitfähigen Werkstoffen vermieden hat. Die paradox erscheinende Kombination bewirkt erstmals nicht nur eine gute Wärmespeicherung, sondern gleichzeitig eine gute flächenmäßige Verteilung der Wärme. Dadurch werden lokale Überhitzung und lokale Unterkühlung vermieden bzw. rasch ausgeglichen. Bei den erfindungsgemäß verbesserten Einlegesohlen wird dieser Effekt noch verstärkt durch die hautverträgliche Stoffschicht (S), die zusammen mit der Schicht (3) genoppt und daher besonders stark verpresst ist, die Luft- und Feuchtigkeitszirkulation im Bereich der Noppung sowie die orthopädische Verformung im Mittelfuß- und Fersenbereich der Laminate. Die erfindungsgemäß verringerte Wärmeisolierung im Vorderfußbereich wird überraschenderweise durch die gute Wärmeleitfähigkeit der Metallschicht kompensiert. Die fläche Verpressung im Vorderfußbereich ermöglicht es, insbesondere für Damenschuhe besonders dünne Einlegesohlen herzustellen.

Selbstverständlich ist es möglich, diese Einlegesohlen auch zu dauerhaften Bestandteilen von Sohlen zu machen. Hierzu werden sie vorgefertigt mit den Sohlen herkömmlicher Schuhe, Stiefel, Sandalen etc. verklebt. Da Polyethylenschaumstoff sich im allgemeinen nur relativ schwer dauerhaft verkleben läßt, empfiehlt es sich auch die Unterseite derartiger Bestandteile von Sohlen mit einer Stoffschicht zu kaschieren und diese dann mit der Innensohle des Schuhwerks zu verkleben.

Die Herstellung der erfindungsgemäßen Einlegesohlen und Bestandteilen von Sohlen erfolgt vorzugsweise dadurch, daß man die hautverträgliche Stoffschicht sowie gegebenenfalls die auf der Unterseite zur Klebung von Sohlenbestandteilen gewünschte Stoffschicht vorher mit der Polyethylenschaumstoffschicht verklebt. Dies erfolgt in besonders einfacher Weise durch Flammkaschieren. Selbstverständlich ist es jedoch auch möglich, diese Stoffe mit Klebstoffen aufzubringen. Hierfür eignen sich insbesondere Thermokleber, die eine ausreichende Elastizität und Dauerbelastbarkeitaufweisen.

Die Verklebung zwischen den Polethylenschaumstoffschichten und der Metallschicht erfolgt vorzugsweise dadurch, daß man handelsübliche selbstklebende Polyethylenschaumstoffschichten mit der Metallfolie verklebt. Prinzipiell ist es aber auch möglich, diese Verklebung mit elastischen Thermoklebern durchzuführen.

Nachdem die vorgefertigten Laminate aus den Schichten (1), (2), (3) und (S) hergestellt sind, werden sie, vorzugsweise in einem Arbeitsgang bei, Temperaturen von 120 bis 170°C unter Druck verformt, verpresst und genoppt. Prinzipiell wäre es zwar auch möglich, von vorher genopptem Material auszugehen, jedoch würde bei der anschließenden orthopädischen Verformung und flachen Verpressung im Vorderfußbereich die Noppung zumindest teilweise wieder verloren gehen. Im Gegensatz zu herkömmlichen Einlegesohlen sind die erfindungsgemäßen Einlegesohlen nicht mehr flach, sondern orthopädisch verformt und vorzugsweise sogar gesprengt.

Eine bevorzugte Ausführungsform der erfindungsgemäß verbesserten Einlegesohle ist in der anliegenden Figur näher erläutert.
Hierin bedeuten:

1  die PE-Schaumstoffschicht,
2  die Metallschicht,
3  eine weitere PE-Schaumstoffschicht,
S  eine Stoffschicht.

Die großen Pfeile im Fersen- und Mittelfußbereich deuten den Wärmefluß vom Fuß in die Einlegesohle an. Die kleineren Pfeile im Vorderfußbereich deuten, daß dort Wärme von der Einlegesohle an den Fuß abgegeben wird.

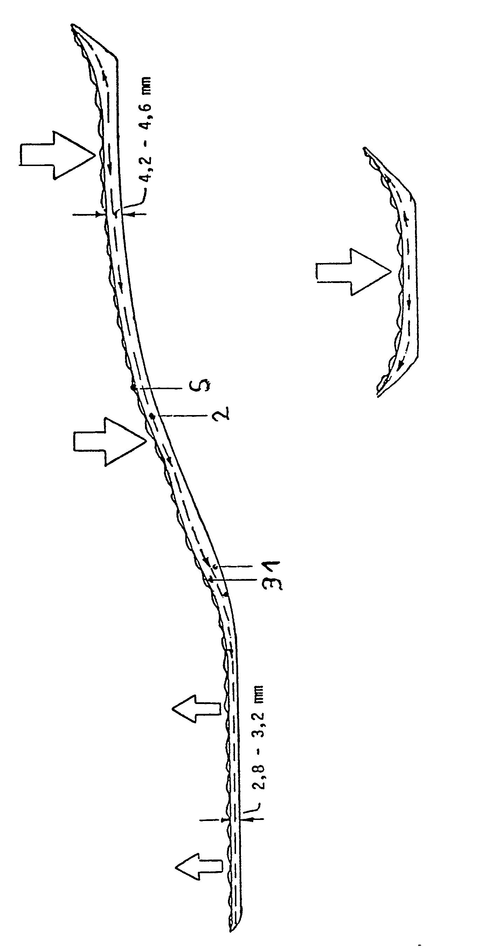

Die Figur stellt einen Längs- und Querschnitt durch die Sohle dar. Die dem Fuß zugewandte Oberfläche ist vorzugsweise quadratisch oder rombenförmig genoppt, und zwar in Abständen von ca. 5 mm durch keilförmige Vertiefungen von ca. 0,5 bis 1 mm.

Als Ausgangsmaterial wurden für die Polyethylenschaumstoffschichten (1) und (3) 3 mm starke selbstklebende Schichten verwendet, und zwar Polyethylenschaum Nr. 1503 der Firma Alveo mit einem ursprünglichen Raumgewicht von 67 kg/m³. Bei der fertigen Einlegesohle beträgt die Dicke des Gesamtlaminats im Vorderfußteil noch 2,8 bis 3,2 mm und im Fersenteil 4,2 bis 4,6 mm. In den Kanten- und Randzonen beträgt die Dicke der Polethylenschaumstoffschicht (1) sogar mehr als 3 mm.

Aus dem Längsschnitt sieht man, das die Einlegesohle S-förmig gekrümmt ist und damit gesprengt ist.

Aus dem Querschnitt sieht man, daß die untere Schaumstoffschicht (1) glatt ist und sich gut an die Sohle eines Schuhs anpassen kann. Die Seitenkanten der Einlegesohle sind hingegen nach oben hochgezogen und verjüngen sich keilförmig, so daß sie orthopädisch verformt ein besonders angenehmes Tragegefühl vermitteln.

Die Metallschicht (2) besteht bei dieser Ausführungsform aus einer 40 µm starken Aluminiumfolie. Diese kann gewünschtenfalls perforiert sein, um beim Verformen unter Druck und Wärme einen Gasaustausch zu ermöglichen. Obendrein können selbstverständlich auch die Schaumstoffschichten (1) und (3) perforiert sein.

## Patentansprüche

1. Einlegesohlen und Bestandteile von Sohlen mit einer zwischen zwei Kunststoffschichten (1, 3) eingebetteten Metallschicht (2) sowie einer hautverträglichen Stoffschicht (S), bei denen alle Schichten fest miteinander laminiert sind, *dadurch gekennzeichnet, daß*

- die Kunstoffschichten aus geschlossenporigem Polyethylenschaumstoff bestehen und ursprünglich - d.h. in unverpreßtem Zustand - je 2 bis 8 mm stark sind;

- die Schaumstoffschichten zur orthopädischen Formgebung mindestens im Vorderfußbereich flach verpreßt sind, wobei die Dicke der Einlegesohlen bzw. der Bestandteile von Sohlen im Vorderfußbereich geringer ist als im Mittelfuß- und Fersenbereich;
- die Metallschicht 0,02 bis 0,08 mm stark ist; und
- im Bereich der Kunststoffschicht (3) und der hautverträglichen Stoffschicht (S) eine Noppung vorgesehen ist.

2. Einlegesohlen und Bestandteile von Sohlen gemäß Anspruch 1 *dadurch gekennzeichnet, daß* die Schaumstoffschichten nach dem Verformen, Verpressen und Noppen noch 20 bis 80 % der ursprünglichen Dicke aufweisen.

3. Einlegesohlen und Bestandteile von Sohlen gemäß Anspruch 1 oder 2, *dadurch gekennzeichnet, daß* die Metallschicht aus Aluminium besteht.

4. Einlegesohlen und Bestandteile von Sohlen gemäß einem der Ansprüche 1 bis 3, *dadurch gekennzeichnet, daß* die hautverträgliche Außenschicht aus Baumwolltrikot besteht.

5. Einlegesohlen und Bestandteile von Sohlen gemäß einem der Ansprüche 1 bis 4, *dadurch gekennzeichnet, daß* sie gesprengt sind.

6. Verfahren zur Herstellung von Einlegesohlen und Bestandteilen von Sohlen gemäß einem der Ansprüche 1 bis 5, *dadurch gekennzeichnet, daß* die Schichten fest laminiert sind und bei Temperaturen von 120 bis 170°C unter Druck im Mittelfuß- und Fersenbereich orthopädisch verformt, im Vorderfußbereich flach verpreßt und im Bereich der Schichten (3) und (S) genoppt werden, so daß sie nach dem Verformen, Verpressen und Noppen 20 bis 80 % der ursprünglichen Dicke aufweisen.

## Claims

1. Insoles and components of soles comprising a metal layer (2) embedded between two layers (1, 3) of a synthetic material and a skin-compatible layer of a textile fabric (S), wherein all of the layers have been firmly laminated to one another, *characterized in that,*

- the layers of a synthetic material consist of a closed-porous polyethylene foam having a thickness of initially - i.e. in the unmoulded state - from 2 to 8 mm;
- for orthopaedic moulding the foam layers have been flatly compressed in the forefoot region, whereby the thickness of the insoles and/or components of soles, respectively, is lower in the forefoot region than in the middle foot and heel regions;
- the metal layer has a thickness of from 0.02 to 0.08 mm; and
- naps have been provided in the regions of the layer (3) of a synthetic material and the skin-compatible layer of a textile fabric (S).

2. Insoles and components of soles according to claim 1, *characterized in that* the foam layers, upon having a been moulded, compressed and napped, still have a thickness of from 20 to 80 % of the initial thickness.

3. Insoles and components of soles according to claims 1 or 2, *characterized in that* the metal layer consists of aluminum.

4. Insoles and components of soles according to any one of claims 1 to 3, *characterized in that* the skin-compatible outer layer consists of cotton tricot.

5. Insoles and components of soles according to any one of claims 1 to 4, *characterized in that* they are broken up.

6. A process for manufacturing insoles and components of soles according to any one of claims 1 to 5, *characterized in that* the layers have been laminated to adhere firmly together and are orthopaedically moulded under pressure at a temperature of from 120°C to 170°C in the middle foot and heel regions, are flatly compressed in the forefoot region and are provided with naps in the regions of the layers (3) and (S) so that, after moulding, compressing and napping, they have a thickness of from 20 to 80% of the initial thickness.

## Revendications

1. Semelles orthopédiques et parties constitutives de semelles, comprenant une couche métallique (2) insérée entre deux couches (1, 3) de matière synthétique, et une couche (S) de tissu supportable par la peau, toutes ces couches étant liées ensemble par co-laminage, *caractérisées en ce que* les couches de matière synthétique sont constituées par une couche de mousse de polyéthylène à pores fermés et ont eu primitivement chacune - à l'état non comprimé - une épaisseur de 2 à 8 mm pour le façonnage de la forme orthopédique des couches de matière mousse, celles-ci sont comprimées sous une forme plate, au moins dans la région de la partie avant du pied, l'épaisseur des semelles orthopédiques et des parties constitutives étant plus petite dans la région de la partie avant du pied que dans la région de sa partie moyenne et dans la région du talon la couche métallique a 0,02 à 0,08 mm d'épaisseur et un nopage est prévu dans la région de la couche (3) de matière synthétique et de la couche (S) de matière supportable par la peau.

2. Semelles orthopédiques et parties constitutives de semelles selon la revendication 1, *caractérisées en ce que* les couches de matière mousse présentent après façonnage, compression et nopage, encore 20 à 80 % de leur épaisseur primitive.

3. Parties orthopédiques et constitutives de semelles selon la revendication 1 ou 2, *caractérisée en ce que* la couche métallique est en aluminium.

4. Parties orthopédiques et constitutives de semelles selon l'une des revendications 1 à 3, *caractérisée en ce que* la couche extérieure supportable par la peau est en tricot de coton.

5. Parties orthopédiques et parties constitutives de semelles selon l'une des revendications 1 à 4, *caractérisée en ce qu'elles* sont rehaussées.

6. Procédé pour la fabrication de semelles orthopédiques et parties constitutives de semelles selon l'une des revendications 1 à 5, *caractérisé en ce que* les couches sont laminées solidairement et, à des températures de 120 à 170°C, façonnées sous pression avec forme orthopédique dans la région moyenne du pied et, dans la région du talon, elles sont comprimées avec aplatissement dans la région avant du pied et nopées dans la région des couches (3) et (5), de manière à présenter, après façonnage, compression et nopage, 20 à 80 % de l'épaisseur primitive.

4,2 - 4,6 mm

5

2

31

2,8 - 3,2 mm

1